# EUROPEAN PATENT APPLICATION

(11) **EP 4 796 649 A1**
(43) Date of publication of application: **26.08.2026**
(21) Application number: 25382153.2
(22) Date of filing: 20.02.2025
(51) Int. Cl.: C12Q 1/6886

(54) **IN VITRO METHOD FOR THE DIAGNOSIS AND/OR PROGNOSIS OF PANCREATIC CANCER; AND/OR MONITORING TREATMENT RESPONSE OF PATIENTS SUFFERING FROM PANCREATIC CANCER**

(71) Applicant: Servizo Galego De Saúde, 15703 Santiago de Compostela, A Coruna (ES); Universidade de Santiago de Compostela (USC), 15872 Santiago de Compostela A Coruña (ES); Fundación Pública Galega Instituto de Investigación Sanitaria de Santiago de Compostela, 15706 Santiago de Compostela, A Coruña (ES)
(72) Inventor: DÍAZ LAGARES, Ángel, 15706 Santiago de Compostela (ES); BROZOS VÁZQUEZ, Elena, 15706 Santiago de Compostela (ES); LÓPEZ LÓPEZ, Rafael, 15706 Santiago de Compostela (ES); COSTA FRAGA, Nicolás, 15706 Santiago de Compostela (ES)
(74) Representative: Hoffmann Eitle

(57) **Abstract**

The present invention refers to the medical field. Particularly, the present invention refers to an *in vitro* method for the diagnosis and/or prognosis of pancreatic cancer; and/or monitoring treatment response of patients suffering from pancreatic cancer.

## Description

### FIELD OF THE INVENTION

The present invention refers to the medical field. Particularly, the present invention refers to an *in vitro* method for the diagnosis and/or prognosis of pancreatic cancer (PC); and/or monitoring treatment response of patients suffering from pancreatic cancer.

### STATE OF THE ART

PC is one of the deadliest cancers, ranking as the third leading cause of cancer-related deaths globally. The high mortality rate is primarily due to late-stage diagnoses and the limited efficacy of current treatments. This cancer represents a significant public health challenge, as it is associated with high morbidity due to severe pain, bile duct obstruction, and functional decline, leading to a substantial reduction in patients' quality of life. Consequently, there is a critical need to improve early detection and enhance disease monitoring to assess therapeutic response and/or disease progression using minimally invasive techniques.

Traditionally, the diagnosis and molecular profiling of cancer patients have relied on tissue biopsies. However, in recent years, liquid biopsy, which involves analyzing blood or other body fluids, has emerged as a promising alternative for detailed patient evaluation across various cancer types. One of the main advantages of this technique is its minimal invasiveness, which allows for the early detection of the disease and the real-time study of tumor progression mechanisms and treatment resistances, which can evolve over time. Circulating free DNA (cfDNA) is a relevant component of liquid biopsy, which can exhibit several types of molecular alterations that reflect the tumor's molecular profile, such as epigenetic modifications.

Epigenetic modifications, such as DNA methylation, play a crucial role in regulating the expression of tumor suppressor genes and oncogenes in malignant cells. DNA methylation, the most well-characterized and studied epigenetic modification, involves the addition of methyl groups (CH3) to cytosines (C) in CpG dinucleotides of DNA, thus regulating gene expression. Because methylated DNA is released from tumor cells into the bloodstream, analyzing this epigenetic mark in liquid biopsy elements, such as cfDNA, holds significant promise as a biomarker in cancer. In this context, recent studies have shown the clinical utility of cfDNA methylation analysis for early diagnosis and monitoring therapeutic response in certain gastrointestinal cancers, such as colorectal cancer.

Early diagnosis of PC remains a formidable challenge. Currently, no approved biomarkers exist for the early detection of PC, which significantly hampers the ability to diagnose the disease at a stage where curative treatment options are still viable. Most cases are diagnosed at an advanced stage, resulting in poor prognosis and limited treatment options. The development of reliable biomarkers for early detection would represent a significant breakthrough in improving the survival rates for PC patients. Liquid biopsy, particularly the analysis of cfDNA methylation, holds potential for the early detection of PC by identifying epigenetic changes that occur in the early stages of tumor development.

The therapeutic management of PC represents a significant challenge for oncologists. Current follow-up tools, including symptom-based assessments, changes in tumor markers like CEA or CA19.9, and radiological findings, are often insufficiently sensitive and specific for evaluating the treatment efficacy of this tumor type. Discontinuing ineffective treatments reduces toxicity and allows patients to benefit from more effective therapeutic strategies. Since DNA methylation is a mechanism that contributes to disease progression and can be detected through liquid biopsy studies, monitoring methylation in cfDNA could establish new guidelines for predicting and tracking therapy response, ultimately improving patients' quality and life expectancy.

There is a clinical need for biomarkers that enable early detection and monitoring therapeutic response in PC to achieve better and more personalized patient management. Therefore, this present invention aims to identify and validate epigenetic biomarkers based on methylation as a useful tool for non-invasive early detection and for monitoring therapeutic response and disease progression of metastatic pancreatic adenocarcinoma patients.

### DESCRIPTION OF THE INVENTION

### Brief description of the invention

The present invention refers to an *in vitro* method identifying biomarker signatures for the diagnosis and/or prognosis of pancreatic cancer; and/or monitoring treatment response of patients suffering from pancreatic cancer. Particularly, the present invention refers to an *in vitro* method for the diagnosis and/or prognosis of pancreatic cancer; and/or monitoring treatment response of patients suffering from pancreatic cancer.

Such as it is shown in the Examples set below, the inventors of the present invention have identified methylation biomarkers in primary tumors of PC and have validated signatures comprising at least two of the following genes: *AJAP1, PRKCB* and *WBSCR17* for the non-invasive detection of PC. Although the best statical results are provided by gene combinations comprising at least two of the above cited genes, the Examples set below also show that the genes have individually given a high AUC value so any of them could be individually used for the diagnosis and/or prognosis of PC; and/or for monitoring therapeutic response and disease progression of patients suffering from PC.

So, the first embodiment of the present invention refers to an *in vitro* method for identifying biomarker signatures for the diagnosis and/or prognosis of PC, or a precancerous stage thereof; and/or monitoring treatment response of patients suffering from PC, or a precancerous stage thereof, wherein the method comprises assessing the methylation status of at least two genes selected from the list consisting of: *AJAP1, PRKCB* and/or *WBSCR17,* in a biological sample obtained from a subject; or to the *in vitro* use of at least two genes selected from the list consisting of: *AJAP1, PRKCB* and/or *WBSCR17,* or of a kit comprising reagents for the determining the methylation status the genes, for identifying biomarker signatures for the diagnosis and/or prognosis of PC, or a precancerous stage thereof; and/or monitoring treatment response of patients suffering from PC, or a precancerous stage thereof.

The second embodiment of the present invention refers to an *in vitro* method for the diagnosis and/or prognosis of PC, or a precancerous stage thereof; and/or monitoring treatment response of patients suffering from PC, or a precancerous stage thereof, wherein the method comprises assessing the methylation status of at least a gene selected from the list consisting of: *AJAP1* and/or *WBSCR17,* in a biological sample obtained from a subject; or to the *in vitro* use of at least one gene selected from the list consisting of: *AJAP1* and/or *WBSCR17,* or of a kit comprising reagents for the determining the methylation status the genes, for the diagnosis and/or prognosis of PC, or a precancerous stage thereof; and/or monitoring treatment response of patients suffering from PC, or a precancerous stage thereof.

In a preferred embodiment, the *in vitro* method for the diagnosis and/or prognosis of PC, or a precancerous stage thereof; and/or monitoring treatment response of patients suffering from PC, or a precancerous stage thereof, further comprises assessing the methylation status of the gene *PRKCB* in a biological sample obtained from the patient.

In a preferred embodiment, the method of the invention comprises assessing the methylation status of the following gene combinations: *AJAP1 + PRKCB; AJAP1 + WBSCR17; PRKCB + WBSCR17; or AJAP1 + PRKCB + WBSCR17.*

In a preferred embodiment, if a deviation of the methylation status is identified, as compared with a pre-established reference value, this is indicative that the biomarker signature may be used for the diagnosis and/or prognosis of PC, or a precancerous stage thereof; and/or monitoring treatment response of patients suffering from PC, or a precancerous stage thereof, or is indicative that the subject is suffering from PC, or a precancerous stage thereof, or has a poor prognosis.

In a preferred embodiment, the methylation status of the genes is determined in at least a CpG site of the gene.

In a preferred embodiment, the methylation status of the genes is determined in at least a CpG site of the promoter region.

In a preferred embodiment, the methylation status of the gene *AJAP1* is determined in at least the CpG site cg13495205, the methylation status of the gene *PRKCB* is determined in at least the CpG site cg03306374 and/or the methylation status of the gene *WBSCR17* is determined in at least a CpG site selected from cg03044249 or cg01366419 (see **Table 1**).

**Table 1**

| **Gene** | **CG ID (EPIC)** | **CHR** | **POS** | **HUMAN GENOME VERSION** | **PROMOTE R** |
|---|---|---|---|---|---|
| *AJAP1* | - | 1 | 4713990 | Human GRCh37/hg19 | Yes |
| | - | 1 | 4713997 | Human GRCh37/hg19 | Yes |
| | cg13495205 | 1 | 4714033 | Human GRCh37/hg19 | Yes |
| | - | 1 | 4714040 | Human GRCh37/hg19 | Yes |
| | - | 1 | 4714081 | Human GRCh37/hg19 | Yes |
| | - | 1 | 4714084 | Human GRCh37/hg19 | Yes |
| PRKCB | - | 16 | 23847297 | Human GRCh37/hg19 | Yes |
| | - | 16 | 23847307 | Human GRCh37/hg19 | Yes |
| | cg03306374 | 16 | 23847325 | Human GRCh37/hg19 | Yes |
| | - | 16 | 23847338 | Human GRCh37/hg19 | Yes |
| | - | 16 | 23847386 | Human GRCh37/hg19 | Yes |
| | - | 16 | 23847392 | Human GRCh37/hg19 | Yes |
| WBSCR17 | - | 7 | 70597019 | Human GRCh37/hg19 | Yes |
| | - | 7 | 70597034 | Human GRCh37/hg19 | Yes |
| | cg03044249 | 7 | 70597065 | Human GRCh37/hg19 | Yes |
| | - | 7 | 70597074 | Human GRCh37/hg19 | Yes |
| | - | 7 | 70597079 | Human GRCh37/hg19 | Yes |
| | - | 7 | 70597083 | Human GRCh37/hg19 | Yes |
| | cg01366419 | 7 | 70597091 | Human GRCh37/hg19 | Yes |
| | - | 7 | 70597095 | Human GRCh37/hg19 | Yes |

In a preferred embodiment, the biological sample is selected from a biological fluid obtained from liquid biopsy, such as plasma, serum, blood, saliva, cerebrospinal fluid or urine, cyst fluid, pancreatic juice or duodenal juice; or a tissue sample; preferably a minimally invasive sample.

The third embodiment of the present invention refers to a kit, suitable for the diagnosis and/or prognosis of PC, or a precancerous stage thereof; and/or monitoring treatment response of patients suffering from PC, or a precancerous stage thereof, which comprises a pair of primers and a probe for the amplification and detection of at least a fragment of gene *AJAP1* and/or a pair of primers and a probe for the amplification and detection of at least a fragment of gene *WBSCR17,* and wherein:
a) The forward primer for the amplification of at least a fragment of gene *AJAP1* is selected from SEQ ID NO: 1 or SEQ ID NO: 4;
b) The reverse primer for the amplification of at least a fragment of gene *AJAP1* is selected from SEQ ID NO: 2 or SEQ ID NO: 5;
c) The probe for the detection of the methylation of the gene *AJAP1* is selected from SEQ ID NO: 3 or SEQ ID NO: 6;
d) The forward primer for the amplification of at least a fragment of gene *WBSCR17* is selected from SEQ ID NO: 13 or SEQ ID NO: 16;
e) The reverse primer for the amplification of at least a fragment of gene *WBSCR17* is selected from SEQ ID NO: 14 or SEQ ID NO: 17;
f) The probe for the detection of the methylation of the gene *WBSCR17* is selected from SEQ ID NO: 15 or SEQ ID NO: 18.

In a preferred embodiment, the kit further comprises a pair of primers and a probe for the amplification and detection of gene *PRKCB,* and wherein:
a) The forward primer for the amplification of at least a fragment of gene *PRKCB* is selected from SEQ ID NO: 7 or SEQ ID NO: 10;
b) The reverse primer for the amplification of at least a fragment of gene *PRKCB* is selected from SEQ ID NO: 8 or SEQ ID NO: 11;
c) The probe for the detection of the methylation of the gene *PRKCB* is selected from SEQ ID NO: 9 or SEQ ID NO: 12.

The present invention also refers to a method for treating PC, or a precancerous stage thereof which comprises, as a first step, diagnosis and/or prognosis of PC, or a precancerous stage thereof; and/or for monitoring therapeutic response and disease progression of patients suffering from PC, or a precancerous stage thereof, following the method of the present invention. The main treatments for PC, or a precancerous stage thereof, depend on the stage and type of the cancer, as well as the patient's overall health. The most common treatment options that would be used once the patient is diagnosed with PC, or a precancerous stage thereof are:
1. Surgery:
   - Whipple Procedure (Pancreaticoduodenectomy): This is the most common surgery for tumors in the head of the pancreas.
   - Distal Pancreatectomy: Removes the tail and sometimes part of the body of the pancreas.
   - Total Pancreatectomy: Involves the removal of the entire pancreas, though it's less common.
2. Radiation Therapy:
   - Often used to shrink tumors before surgery or to kill any remaining cancer cells after surgery.
   - External beam radiation therapy is the most common type.
3. Chemotherapy:
   - Drugs like gemcitabine or combinations such as FOLFIRINOX (a combination of fluorouracil, leucovorin, irinotecan, and oxaliplatin) are frequently used.
   - Chemotherapy may be used before surgery (neoadjuvant) or after surgery (adjuvant) to improve outcomes.
4. Targeted Therapy:
   - Drugs that target specific genetic mutations or proteins involved in cancer cell growth.
   - Erlotinib, which targets the EGFR protein, is sometimes used for certain patients.
5. Immunotherapy:
   - Typically used for PC with specific genetic changes, such as mismatch repair deficiency or high microsatellite instability.
   - Checkpoint inhibitors like pembrolizumab can be options for these cases.
6. Palliative Care:
   - Focuses on relieving symptoms and improving quality of life, especially for advanced-stage pancreatic cancer.
   - This might include pain management, nutritional support, and treatment of jaundice (e.g., stent placement for bile duct obstruction).
7. Clinical Trials:
   - Participation in clinical trials can provide access to new and experimental treatments that may be effective when standard therapies are not.

Treatment plans often involve a combination of these approaches, customized to each patient's needs and the cancer's specifics.

In a preferred embodiment, the present invention is a computer-implemented invention, wherein a processing unit (hardware) and a software are configured to: Receive the methylation status of any of the above cited biomarkers or signatures, process the methylation status received for finding substantial variations or deviations, and provide an output through a terminal display of the variation or deviation of the methylation status, wherein the variation or deviation of the methylation status indicates that the subject may be suffering from PC or that the patient has a poor prognosis.

The diagnosis, prognosis or monitoring carried out in the context of the present invention by means of assessing the methylation status of the genes, may be also combined with image techniques. Several imaging techniques are used to diagnose, stage, and monitor treatment response in pancreatic cancer. Here are the main imaging methods that could be used in the context of the present invention:
1. Computed Tomography (CT) Scan:
   - A CT scan is one of the most commonly used imaging techniques for detecting pancreatic cancer.
   - Multiphase CT scans with contrast provide detailed images of the pancreas and surrounding structures, helping determine the size and spread of the tumor.
2. Magnetic Resonance Imaging (MRI):
   - An MRI uses magnetic fields and radio waves to produce detailed images of the pancreas.
   - MRCP (Magnetic Resonance Cholangiopancreatography) is a special type of MRI that focuses on the bile ducts and pancreatic ducts, useful for identifying blockages or abnormalities.
3. Endoscopic Ultrasound (EUS):
   - Combines endoscopy and ultrasound to create detailed images of the pancreas from inside the digestive tract.
   - EUS is highly effective for detecting small tumors and can be used to guide fineneedle aspiration (FNA) for biopsy.
4. Positron Emission Tomography (PET) Scan:
   - Often combined with CT (PET/CT), it uses a radioactive tracer to detect cancerous cells based on their increased glucose metabolism.
   - Useful for assessing the spread (metastasis) of cancer and evaluating treatment response.
5. Ultrasound (Abdominal Ultrasound):
   - A non-invasive test that can sometimes detect pancreatic tumors or cysts but is less detailed than CT or MRI.
   - Often used as an initial screening tool if a patient presents with nonspecific abdominal symptoms.
6. Endoscopic Retrograde Cholangiopancreatography (ERCP):
   - Primarily used to diagnose and treat blockages in the bile or pancreatic ducts.
   - A contrast dye is injected, and X-rays are taken to visualize the ducts. ERCP can also be used to take tissue samples or place stents to relieve blockages.
7. X-rays:
   - Typically, not detailed enough to diagnose PC but may be used in conjunction with other imaging to assess complications, such as bowel obstruction.
8. Laparoscopy:
   - A minimally invasive surgical procedure that allows direct visualization of the pancreas and surrounding areas.
   - Used for staging the cancer to check for metastases not detected by non-invasive imaging.

Each imaging technique has specific benefits and is chosen based on the clinical context, such as initial diagnosis, staging, or treatment planning.

For the purpose of the present invention the following terms are defined:
- "CpG site": The CpG sites refers to dinucleotides formed by a cytosine followed by a guanine. Cytosines in CpG dinucleotide can be methylated to form 5-methylcytosines.
- "CpG island" (CGI): CpG islands (or CG islands) are regions with a high frequency of CpG sites. The usual formal definition is a region with at least 200 bp and a GC percentage greater than 50%.
- "Beta-value": The beta-value provides a quantitative measure of methylation at a specific locus. It is calculated as the ratio of methylated signal intensity to the total signal intensity (M/M+U) at a specific cytosine site. Its value ranges from 0 (unmethylated) to 1 (fully methylated).
- "Bisulfite treatment": Treatment of DNA with sodium bisulfite converts unmethylated cytosine to uracil, which is subsequently converted to thymine during PCR amplification, while methylcytosine remains unchanged, as cytosine.
- "Promoter region": The promoter region of the gene comprises the sequences of the 1500 bp upstream of the transcription start site (TSS), 5'UTR and the first exon of the gene.
- As used herein, the term "methylation" will be understood to mean the presence of a methyl group added by the action of a DNA methyl transferase enzyme to a cytosine base followed by a guanine (CpG) nucleotide of a nucleic acid.
- Accordingly, the term, "methylation status" as used herein refers to the presence or absence of methylation in a specific nucleic acid region.
- The expression "higher level of methylation" refers to an increase in the relative amount of methylation of a nucleic acid, as compared with the subject used as control that, in this case, are healthy subjects or subjects not suffering from PC. Thus, in the present disclosure, the "higher level of methylation" is generally determined with reference to a baseline level represented by the methylation status of a given genomic region in a sample obtained from control subjects. For example, "higher level of methylation" may be at least 2% greater than the baseline level of methylation, for example at least 5% greater than the baseline level of methylation, or at least 10% greater than the baseline level of methylation, or at least 15% greater than the baseline level of methylation, or at least 20% greater than the baseline level of methylation, or at least 25% greater than the baseline level of methylation, or at least 30% greater than the baseline level of methylation, or at least 40% greater than the baseline level of methylation, or at least 50% greater than the baseline level of methylation, or at least 60% greater than the baseline level of methylation, or at least 70% greater than the baseline level of methylation, or at least 80% greater than the baseline level of methylation, or at least 90% greater than the baseline level of methylation.
- The term "comprising" means "including", but not limited to what follows the term "comprising". Thus, the use of the term "comprising" indicates that the elements listed are necessary or mandatory, but that other elements are optional and may or may not be present.
- The term "consisting of" means "including" but is limited to what follows the term "consisting of". Thus, the term "consists of" indicates that the elements listed are mandatory, and that other elements may not be present.
- By "pancreatic cancer" it is understood any stage of this disease, including precancerous conditions of the pancreas, comprising changes to pancreas cells that make them more likely to develop into cancer. These conditions are not yet cancer but it there are not treated there is a chance that these abnormal changes may become pancreatic cancer.
- The expression "liquid biopsy" refers to any sample of biologic fluids that may contain tumor-derived material. Particularly, a liquid biopsy is any sample of biologic fluids (for example: plasma, serum, blood, saliva, cerebrospinal fluid or urine, cyst fluid, pancreatic juice or duodenal juice) that may contain tumor-derived material, such as circulating tumor DNA.
- The expression "minimally invasive biological sample" refers to any sample which is taken from the body of the patient without the need of using harmful instruments, other than fine needles used for taking the blood from the patient, and consequently without being harmfully for the patient. Specifically, minimally invasive biological sample refers in the present invention to blood, serum, or plasma samples.

### Brief description of the figures

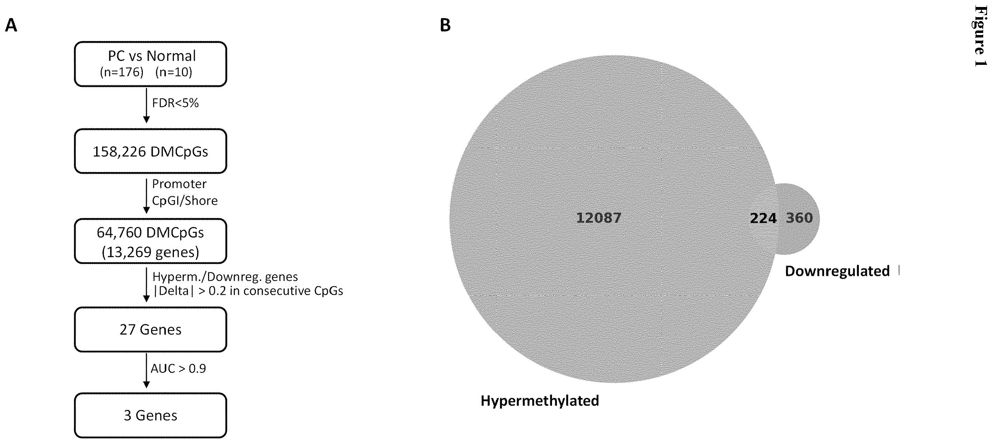

**Figure 1****. Identification of methylation biomarkers in pancreatic cancer. (A)** Workflow for the discovery of methylation biomarkers in primary PC tumors. (**B**) Venn diagram showing the overlap between hypermethylated and downregulated biomarkers in primary PC tumors.

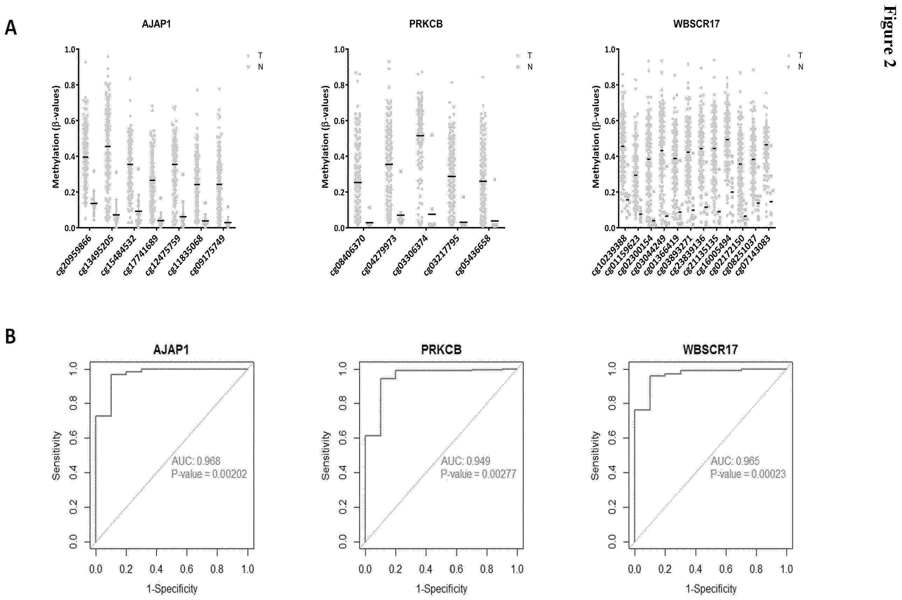

**Figure 2****. Promoter hypermethylation of *AJAP1*, *PRKCB,* and *WBSCR17* in tissue samples of primary pancreatic cancer.** (**A**) DNA methylation levels at the promoters of *AJAP1, PRKCB,* and *WBSCR17* in primary pancreatic tumors (T) compared to adjacent non-tumoral pancreatic tissues (N). (**B**) Receiver Operating Characteristic (ROC) curves showing the diagnostic performance of promoter hypermethylation of *AJAP1, PRKCB,* and *WBSCR17* for detecting pancreatic cancer. AUC, Area under de ROC curve.

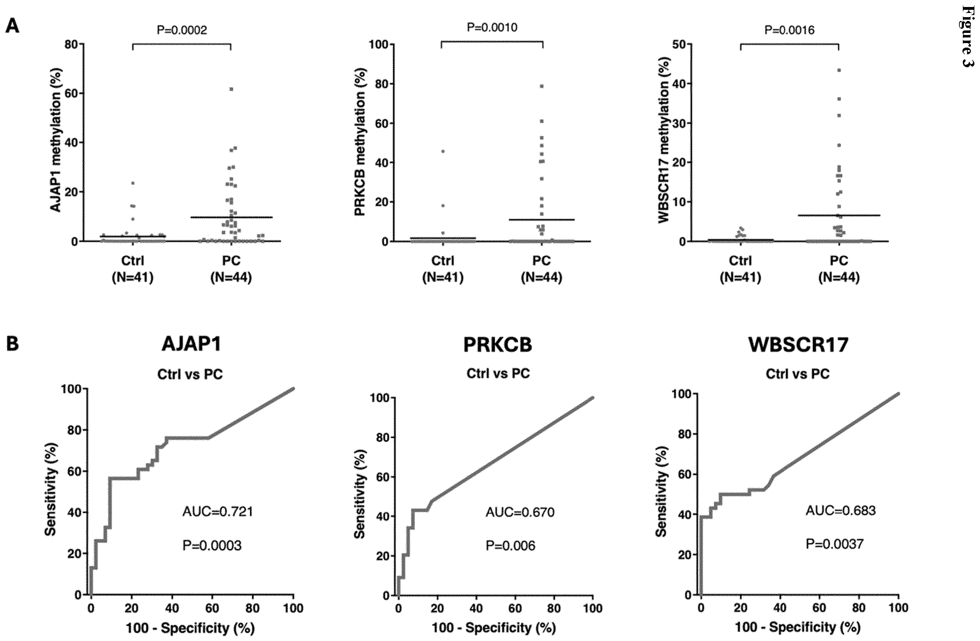

**Figure 3****. Validation of methylation status and diagnostic potential of *AJAP1, PRKCB,* and *WBSCR17* in cfDNA.** (**A**) DNA methylation levels of *AJAP1, PRKCB,* and *WBSCR17* in cfDNA from 44 PC patients compared to 41 healthy controls. (**B**) Receiver Operating Characteristic (ROC) curves showing the diagnostic performance of promoter hypermethylation of *AJAP1, PRKCB,* and *WBSCR17* in cfDNA for detecting pancreatic cancer. PC, pancreatic cancer; Ctrl, controls; AUC, Area under de ROC curve.

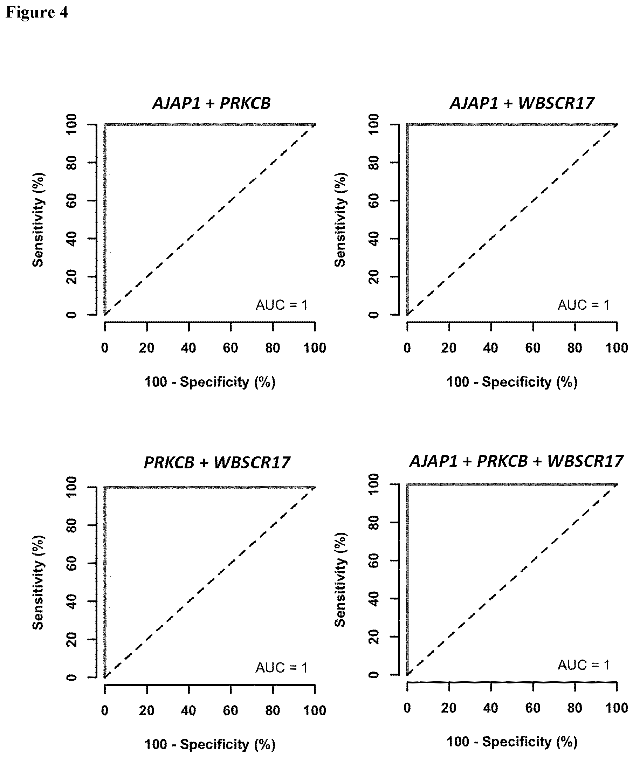

**Figure 4****. Diagnostic performance of combining the promoter methylation of AJAP1, PRKCB, and WBSCR17 for PC detection in tissue samples.** ROC curves show the AUC for detecting PC in tissue samples using the combined promoter methylation of AJAP1, PRKCB, and WBSCR17 in a cohort from TCGA (176 primary tumor tissue samples and 10 adjacent healthy tissue samples from adenocarcinoma PC patients). AUC, Area under the ROC curve.

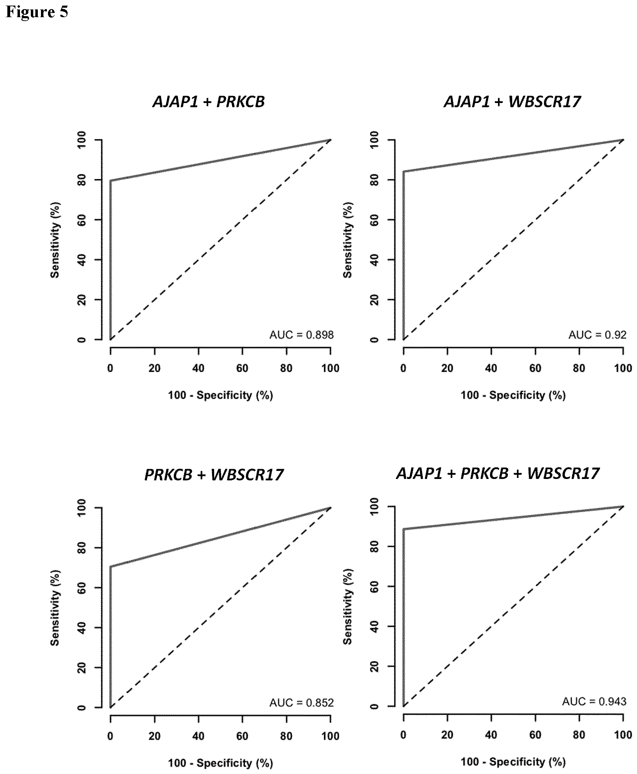

**Figure 5****. Combined promoter methylation analysis of *AJAP1, PRKCB,* and *WBSCR17* in cfDNA for detecting pancreatic cancer.** The methylation data of *AJAP1, PRKCB,* and *WBSCR17* were combined and analyzed using a random forest algorithm in a cohort of 44 PC patients and 41 healthy controls. Receiver Operating Characteristic (ROC) curves shows the diagnostic performance of this combination. AUC, Area under de ROC curve.

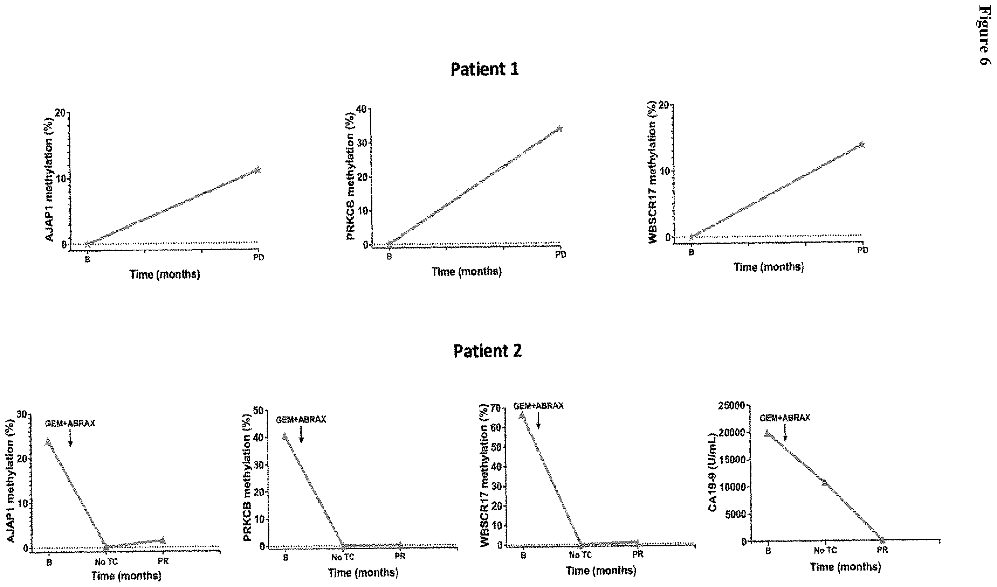

**Figure 6****. Promoter methylation of *AJAP1, PRKCB,* and *WBSCR17* in cfDNA for monitoring treatment response in metastatic pancreatic cancer.** Promoter methylation levels of *AJAP1, PRKCB,* and *WBSCR17* in cfDNA, analyzed by ddPCR at different time points during the disease course in a subgroup of metastatic PC patients. GEM, gemcitabine; ABRAX, abraxane; No CT, computed tomography scan not performed; B, baseline; PD, progressive disease; PR, partial response.

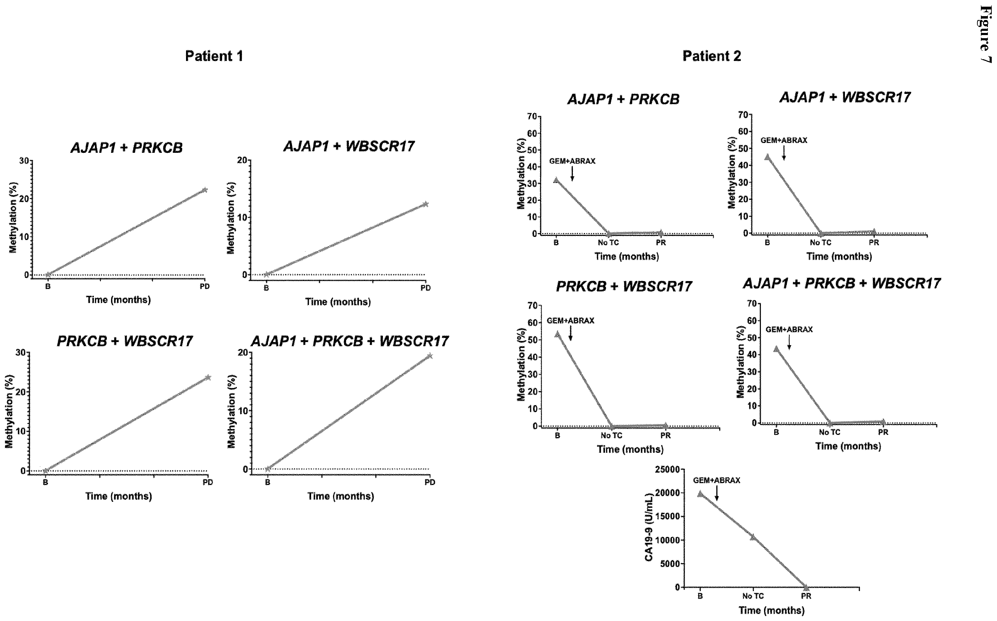

**Figure 7****. Combined promoter methylation of *AJAP1, PRKCB,* and *WBSCR17* in cfDNA for monitoring treatment response in metastatic pancreatic cancer.** Promoter methylation levels of *AJAP1, PRKCB,* and *WBSCR17* in cfDNA, were analyzed by ddPCR at different time points during the disease course in a subgroup of metastatic PC patients. For each longitudinal time point, the methylation average of the combined biomarkers is represented. GEM, gemcitabine; ABRAX, abraxane; No CT, computed tomography scan not performed; B, baseline; PD, progressive disease; PR, partial response.

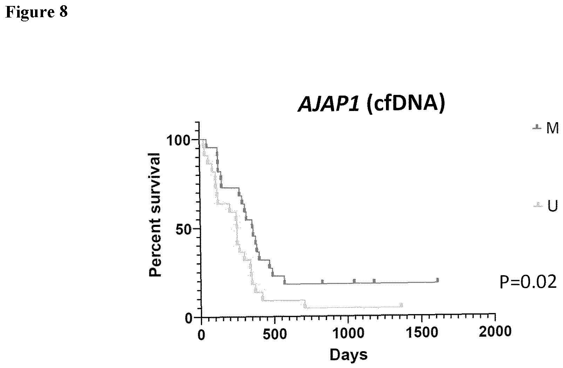

**Figure 8****. Prognostic value of promoter methylation of *AJAP1* in cfDNA of PC patients.** Kaplan-Meir survival curves of PC patients stratified by promoter methylation status of *AJAP1* in cfDNA. Patients with methylation levels above the median were classified as methylated (M), while those below the median were classified as unmethylated (U). The significance of the Kaplan-Meir curve was evaluated by log-rank test.

### Detailed description of the invention

The present invention is illustrated by means of the Examples set below without the intention of limiting its scope of protection.

### Example 1. Materials and methods

### Example 1.1. Genome-wide DNA methylation and transcriptomic analysis in pancreatic primary tumors from the retrospective cohort

Data from genome-wide DNA methylation analysis were sourced from The Cancer Genome Atlas (TCGA) database. IDAT files from the Infinium Human Methylation 450K BeadChip (450K) analysis from 176 primary tumor tissue samples and 10 adjacent healthy tissue samples from adenocarcinoma PC patients were downloaded using the TCGAbiolinks R package. To obtain DNA methylation data, the IDAT files underwent preprocessing, normalization, and quality control using the minfi and limma R packages. To determine the functional relevance of the differentially methylated genes identified in PC, we utilized normalized RNA-sequencing (RNA-seq) expression data from the same cohort of patients, which were also obtained via the TCGAbiolinks R package. Differentially expressed genes were identified using generalized linear models within the package. Criteria for differential expression were set at an FDR < 0.05 and |log2 fold change (FC)| ≥1. Genes differentially expressed between PC and normal tissue samples from TCGA were correlated with the differentially methylated genes using Venn diagrams, created with the VennDiagram R package.

### Example 1.2. Study participants from the prospective cohort

In the prospective cohort of this study (**Table 2**), 44 patients with metastatic PC (mPC) and 41 healthy individuals (controls) were included. Inclusion criteria for the study were to have a recent diagnosis of metastatic or locally advanced pancreatic adenocarcinoma, age over 18 years, not have received any prior treatment with antineoplastic agents in the context of advanced disease. Participants were recruited between 2021 and 2023 at the Medical Oncology Department of the University Clinical Hospital of Santiago de Compostela (CHUS) and the Reina Sofia University Hospital of Córdoba (HURS) in Spain. The study received approval from the Galician Ethical Committee and was conducted following Good Clinical Practice guidelines and the Declaration of Helsinki. All participants provided written informed consent. Patient responses to treatment were monitored using CT scans during follow-up and stablish according to RECIST 1.1 criteria.

**Table 2. Clinical characteristics of patients.**

| **Characteristics** | | **Patients (n=44)** | **Controls (n=41)** |
|---|---|---|---|
| | **Gender** | | |
| Male | | 22 (50%) | 18 (44%) |
| Female | | 22 (50%) | 23 (56%) |
| | **Age (years)** | | |
| Mean ± SD | | 66 ± 10 | 55 ± 12 |
| | **Stage** | | |
| IV | | 44 | - |
| | **Histology** | | |
| Adenocarcinoma | | 44 | - |

| | | | |
|---|---|---|---|
| *SD: Standard deviation* | | | |

### Example 1.3. Blood sample collection and plasma isolation

Serial blood samples were collected in 10 mL Cell-Free DNA BCT collection tubes (Streck, USA) at several time-points: i) just before starting the antineoplastic treatment established by the oncologist, ii) at 4 weeks, iii) at 12 and 24 weeks of treatment (coinciding with evaluation of response by TC scans), iv) as well as at the time of tumor progression. Blood samples were processed within the next 2 hours after collection. Plasma was isolated by an initial centrifugation at 1,600 × g for 10 min at 4°C, followed by a second centrifugation at 16,000 × g for 10 min at 4°C. Isolated plasma was stored at -80°C until use.

### Example 1.4. Extraction and quantification of cfDNA

CfDNA was isolated from 2 mL of plasma using the QIAamp^{®} Circulating Nucleic Acid Kit (Qiagen, Germany) and the QIAvac 24 Plus vacuum system (Qiagen, Germany) following the manufacturer's instructions. CfDNA was eluted in 50 µL of the kit's elution buffer. Concentrations were measured using a Qubit 4.0 Fluorometer (Thermo Fisher Scientific, USA) with the Qubit dsDNA HS Assay Kit (Thermo Fisher Scientific, USA) before storing the samples at -20°C.

### Example 1.5. Bisulfite conversion of cfDNA

Bisulfite conversion of 10-20 ng of cfDNA was carried out using the EZ DNA Methylation-Lightning Kit (Zymo Research, USA) following the manufacturer's recommendations. The bisulfite treatment was conducted using a ProFlex PCR System (Thermo Fisher Scientific, USA) with the following cycling conditions: initial denaturation at 98°C for 8 minutes, followed by incubation at 54°C for 60 minutes and a final hold step of 4°C. The treated cfDNA was then eluted in 10 µL of elution buffer and stored at -20°C.

### Example 1.6. Methylation analysis of cfDNA by ddPCR

The methylation status of *AJAP1, PRKCB,* and *WBSCR17* promoters in cfDNA was analyzed using on a QX200 system (Bio-Rad, USA). Primers for each gene were designed using Methyl Primer Express Software v1.0 (Thermo Fisher Scientific, USA) to detect both methylated (M) and unmethylated (U) sequences (**Table 3**). Probes were manually designed to distinguish between methylated and unmethylated CpGs and verified with OligoEvaluator (Sigma-Aldrich, USA) to prevent duplex formation and secondary structures.

A preamplification reaction was performed initially. For this preamplification, 2 ng of bisulfitetreated cfDNA were mixed with 6.25 µL SsoAdvance PreAmp Supermix (Bio-Rad, USA), 1.25 µL of a primer mix (*AJAP1, PRKCB* and *WBSCR17*), and 3 µL of nuclease-free water, in a final volume of 12.5 µL. PCR conditions were set as follows: 3 minutes at 95°C, followed by 12 cycles of 95°C for 15 seconds and 58°C for 4 minutes, with a final hold at 4°C. The reaction volume was then diluted 1:2 with nuclease-free water.

Next, a multiplex reaction mix was prepared by combining 2 µL of the preamplified product with 11 µL ddPCR Supermix for Probes (No dUTP) (Bio-Rad, USA), 1.1 µL of a primer-probe mix for each gene (at a ratio of 0.400/0.250 µM, respectively), and 7.9 µL of nuclease-free water, in a final volume of 22 µL. Droplets were generated using the QX200^{™} Droplet Generator (Bio-Rad, USA). The thermocycling conditions were: 10 minutes at 95°C, followed by 45 cycles of 94°C for 15 seconds and 54-57°C for 30 seconds, with a final step of 98°C for 10 minutes and a hold at 4°C. The temperature ramp increment was 2.5°C/s for all steps. Droplets were counted and analyzed using the QX200^{™} Droplet Reader (Bio-Rad, USA), and data acquisition was performed with QuantaSoft software (Bio-Rad, USA). Water served as a no-template control, while human methylated and non-methylated DNA (Zymo Research, USA) were used as positive controls for methylation and unmethylation, respectively. Reactions were conducted in duplicate. cfDNA methylation was calculated using the formula: Methylation (%) = [M/(U + M)] × 100, where M represents copies/µL of methylated cfDNA and U represents copies/µL of unmethylated cfDNA.

### Example 1.7. Statistical analysis

To assess methylation differences between groups in TCGA data, hierarchical linear models were employed using RnBeads 2.0. P-values were adjusted for multiple testing with the Benjamini-Hochberg method, setting a threshold of p < 0.05 for statistical significance. For comparing methylation data obtained from ddPCR, the Kolmogorov-Smirnov test was initially used to evaluate the normality of the data distribution. Depending on the results, either the Mann-Whitney U test or the Student's t-test was subsequently applied. A p-value < 0.05 was considered statistically significant.

To evaluate diagnostic accuracy, a receiver operating characteristic (ROC) curve was generated considering the mean methylation levels of the DMCpGs obtained between PC and non-tumor tissues. A Random Forest algorithm was used to determine the diagnostic potential of combined gene data, utilizing the R packages caret, randomForest, and pROC. The utility of biomarkers as prognostic factors was evaluated by Kaplan-Meir curves. Statistical analyses and graphical representations were conducted using GraphPad Prism 8.0 (GraphPad Software, USA) and R statistical software (v4.2.0).

**Table 3. Characteristics of primers and probes for cfDNA methylation analysis.**

| **Assay** | **Primers & Probes** | **Sequence (5'-3')** | **Sequence ID** | **Amplicon¹** | **Position of CpGs²** |
|---|---|---|---|---|---|
| ***AJAP1_M*** | F | GTAGTAGTTGCGTTGGGC TAATAAAATCACCGCGTTC [FAM]TATAGGATCGGGTTTCGTTGGGTT | 1 | chr1:4713980-4714096 | chr1:4713990; |
| | | | | | chr1:4713997 |
| | R | | 2 | | chr1:4714081; |
| | P | | 3 | | chr1:4714084 |
| | | | | | ³chr1:4714033; |
| | | | | | chr1:4714040 |
| ***AJAP1_U*** | F | TAGGTAGTAGTTGTGTTGGGT TAATAAAATCACCACATTCCAA [HEX]TATAGGATTGGGTTTTGTTGGGT | 4 | | chr1:4713990; |
| | | | | | chr1:4713997 |
| | R | | 5 | | chr1:4714081; |
| | P | | 6 | | chr1:4714084 |
| | | | | | ³chr1:4714033; |
| | | | | | chr1:4714040 |
| ***PRKCB_M*** | F | GGTGTTAAGCGTAGTTGGAC CTACTACACCCGAAACCG [FAM]TTGGGCGAGTGATAGTTTCGGT | 7 | chr16:23847288-23847403 | chr16:23847297; |
| | | | | | chr16:23847307 |
| | R | | 8 | | chr16:23847386; |
| | P | | 9 | | chr16:23847392 |
| | | | | | ⁴chr16:23847325; |
| | | | | | chr16:23847338 |
| ***PRKCB_U*** | F | GGTGTTAAGTGTAGTTGGAT CTACTACACCCAAAACCA [HEX]TTGGGTGAGTGATAGTTTTGGTT | 10 | | chr16:23847297; |
| | | | | | chr16:23847307 |
| | R | | 11 | | chr16:23847386; |
| | P | | 12 | | chr16:23847392 |
| | | | | | ⁴chr16:23847325; |
| | | | | | chr16:23847338 |
| ***WBSCR17_M*** | F | GAGCGTTGGTGAGTTTGTC CAACACTAAAAAACGAACGC [FAM]CGGAGAGGACGTAGCGTTC | 13 | chr7:70597016-70597109 | chr7:70597019; |
| | | | | | chr7:70597034 |
| | | | | | ⁵chr7:70597091; |
| | R | | 14 | | chr7:70597095 |
| | P | | 15 | | ⁶chr7:70597065; |
| | | | | | chr7:70597074; |
| | | | | | chr7:70597079; |
| | | | | | chr7:70597083 |
| ***WBSCR17_U*** | F | GTGAGTGTTGGTGAGTTTGTT ACAACACTAAAAAACAAACACC [HEX]TGGAGAGGATGTAGTGTTTGGTT | 16 | | chr7:70597019; |
| | | | | | chr7:70597034 |
| | | | | | ⁵chr7:70597091; |
| | R | | 17 | | chr7:70597095 |
| | P | | 18 | | ⁶chr7:70597065; |
| | | | | | chr7:70597074; |
| | | | | | chr7:70597079; |
| | | | | | chr7:70597083 |

| | | | | | |
|---|---|---|---|---|---|
| *1Chromosomal position, according to hg19, of the DNA fragment amplified and detected. 2 Chromosomal position of the* *CpGs detected, according to hg19, for each primer and probe. 3cg13495205. 4cg03306374. 5cg01366419. 6cg03044249. Abbreviations: M, methylated; U, unmethylated; F, Forward primer; R, Reverse primer; P, Probe.* | | | | | |

### Example 2. Results

### Example 2.1. Identification of Methylation Biomarkers in primary tumors of PC

To identify DMCpGs sites between PC and non-tumoral tissues, we first analysed the methylation data sourced from the TCGA database, encompassing 176 primary PC tissue samples and 10 adjacent non-tumoral pancreatic tissue samples (controls). By employing stringent criteria (FDR < 0.05), we identified 158,226 CpG sites with significant differential methylation between PC and non-tumoral tissues (controls) (**Figure 1A**). Since the hypermethylaton of CpG islands and shores in promoters of tumor suppressor genes is a relevant feature of cancer cells, among the 158,226 DMCpGs, we selected CpG sites only located within CpG islands or shore regions of promoters. This filtering process reduced the number of DMCpG sites to 64,760, corresponding to 13,269 genes (**Figure 1A**). Next, to identify genes that were both hypermethylated and downregulated in PC, we performed an integrative analysis with methylation and gene expression data from the TCGA cohort previously described. Thus, we identified 224 gene candidates that were both hypermethylated and downregulated in PC (**Figure 1B**). To further refine the list of gene candidate, we selected the genes with higher methylation differences between PC and controls (Delta > 0.2) across consecutive CpG sites. This step ensures that the selected genes exhibit consistent and substantial methylation changes across multiple CpG sites, thereby enhancing their reliability as potential biomarkers. This filtering process allowed the selection of 27 candidate genes.

Among the potential 27 candidates, we finally selected the 3 genes (*AJAP1, PRKCB,* and *WBSCR17*) with highest differences in methylation levels between PC and non-tumor tissues (**Figure 2A**) and highest AUCs in ROC curve analyses for the detection of PC (**Figure 2B**).

### Example 2.2. Validation and diagnostic potential of the promoter methylation of AJAP1, PRKCB and WBSCR17 for the non-invasive detection of PC

To validate the methylation status of the 3 candidate genes (*AJAP1*, *PRKCB,* and *WBSCR17*) in cfDNA and to evaluate their diagnostic potential in PC, we selected a cohort of 41 healthy individuals (controls) and 44 PC patients. Importantly, the cfDNA methylation analysis in this cohort confirmed the results previously obtained in primary tumors of PC, showing significantly higher methylation levels in PC than in controls (**Figure 3A**), as well as significant AUCs for the detection of PC (AUC*_{AJAP1}* = 0.721, AUC*_{PRKCB} =* 0.670, AUC*_{WBSCR17} =* 0.683) (**Figure 3B**).

### Example 2.3. Diagnostic utility of combined promoter methylation of AJAP1, PRKCB, and WBSCR17 for detecting PC.

### DNA methylation analysis in tissue samples

To explore whether combining the methylation profiles of the previously analyzed genes (AJAP1, PRKCB, WBSCR17) in DNA from tissue samples could enhance their individual diagnostic accuracy for PC detection, we performed a random forest algorithm on the TCGA cohort: 176 primary tumor tissue samples and 10 adjacent healthy tissue samples from adenocarcinoma PC patients. This analysis revealed that the combination of at least two of these genes resulted in higher AUC values (AUCs=1.0) for PC detection (**Figure 4**) compared to each gene alone (**Figure 2B**). Specifically, the diagnostic accuracy was improved by the methylation status of the following combination of genes: *AJAP1 + PRKCB; AJAP1 + WBSCR17; PRKCB + WBSCR17*; or *AJAP1 + PRKCB + WBSCR17.*

### CfDNA methylation analysis in plasma samples

We also assessed in plasma cfDNA whether the diagnostic accuracy of the three individual genes previously analyzed (AJAP1, PRKCB, WBSCR17) could be enhanced by combining the methylation status of at least two of these genes. Using a random forest algorithm on our cohort of plasma samples (41 controls vs. 44 PC cases), we found that combining at least two genes yielded higher AUCs for PC detection (**Figure 5**) compared to the AUCs of individual genes (**Figure 3B**). These findings indicate that the detection of PC is improved by the methylation status of the following combination of genes: *AJAP1 + PRKCB; AJAP1 + WBSCR17; PRKCB + WBSCR17;* or *AJAP1 + PRKCB + WBSCR17.* Notably, the simultaneous combination of the three genes (*AJAP1 + PRKCB + WBSCR17*) produced the highest diagnostic performance (AUC of 0.943), with high sensitivity (89%) and specificity (100%).

### Example 2.4. Clinical utility of the promoter hypermethylation of AJAP1, PRKCB and WBSCR17 for the non-invasive monitoring of PC

To evaluate the clinical utility of *AJAP1, PRKCB,* and *WBSCR17* for non-invasive monitoring of treatment response in metastatic PC patients, we analyzed the methylation status of each of these 3 genes in cfDNA by ddPCR at different time points of the disease in a subgroup of patients from our previous cohort, whose disease evolution was evaluated according to the standard clinical practice using TC scans. Of note, the cfDNA methylation of *AJAP1, PRKCB* and *WBSCR17* increased (**Figure 5****, Patient 1**) with the progression of the disease and decreased in response to effective therapy (**Figure 5****, Patient 2**), being this decrease faster than the current clinical biomarker CA19-9. These results represent a proof of concept of the utility of evaluating the methylation of these 3 genes as potential non-invasive biomarkers to monitor the disease during the follow-up of metastatic PC patients.

### Example 2.5. Clinical utility of combined promoter methylation of AJAP1, PRKCB and WBSCR17 for the non-invasive monitoring of PC

To evaluate the clinical utility of combining the methylation status of *AJAP1, PRKCB,* and *WBSCR17* for non-invasive monitoring of treatment response in metastatic PC patients, we analyzed the methylation status of these 3 genes in cfDNA by ddPCR at different time points of the disease in two patients of our cohort of plasma samples. Thus, for each longitudinal time point, the methylation average of the combined biomarkers was calculated. The evolution of disease was evaluated according to standard clinical practice using TC scans. Of note, the combined methylation status of at least two of the three genes analyzed in cfDNA (*AJAP1 + PRKCB; AJAP1 + WBSCR17*; *PRKCB + WBSCR17;* or *AJAP1 + PRKCB + WBSCR17*) increased with the progression of the disease (**Figure 6****, Patient 1**) and decreased in response to effective therapy (**Figure 6****, Patient 2**). This reduction occurred faster than the decrease observed in CA19-9 serum levels, which is the standard biomarker used in the clinic to monitor the evolution of the PC disease. These results confirm the utility of combining the methylation status of at least two of these 3 genes as non-invasive biomarkers to monitor the disease during the follow-up of PC patients.

### Example 2.6. Utility of promoter methylation of AJAP1, PRKCB and WBSCR17 in cfDNA as a prognostic biomarker in PC

To evaluate the utility of *AJAP1, PRKCB* and *WBSCR17* in cfDNA as prognostic factors of PC, we analyzed the individual (*AJAP1, PRKCB,* or *WBSCR17*) and combined (*AJAP1 + PRKCB; AJAP1 + WBSCR17*; *PRKCB + WBSCR17;* or *AJAP1 + PRKCB + WBSCR17*) promoter methylation of these genes in the plasma cohort of PC patients (n=44) of our study. This analysis indicated that only the promoter methylation *AJAP1* in cfDNA, was significantly associated with poorer survival outcomes in PC (**Figure 7**).

## Claims

1. *In vitro* method for identifying biomarker signatures for the diagnosis and/or prognosis of pancreatic cancer, or a precancerous stage thereof; and/or monitoring treatment response of patients suffering from pancreatic cancer, or a precancerous stage thereof, wherein the method comprises assessing the methylation status of at least two genes selected from the list consisting of: *AJAP1, PRKCB* and/or *WBSCR17,* in a biological sample obtained from a subject.

2. *In vitro* method for the diagnosis and/or prognosis of pancreatic cancer, or a precancerous stage thereof; and/or monitoring treatment response of patients suffering from pancreatic cancer, or a precancerous stage thereof, wherein the method comprises assessing the methylation status of at least a gene selected from the list consisting of: *AJAP1* and/or *WBSCR17,* in a biological sample obtained from a subject.

3. *In vitro* method for the diagnosis and/or prognosis of pancreatic cancer, or a precancerous stage thereof; and/or monitoring treatment response of patients suffering from pancreatic cancer, or a precancerous stage thereof, according to claim 2, wherein the method further comprises assessing the methylation status of the gene *PRKCB* in a biological sample obtained from the patient.

4. *In vitro* use of at least two genes selected from the list consisting of: *AJAP1, PRKCB* and/or *WBSCR17,* or of a kit comprising reagents for the determining the methylation status the genes, for identifying biomarker signatures for the diagnosis and/or prognosis of pancreatic cancer, or a precancerous stage thereof; and/or monitoring treatment response of patients suffering from pancreatic cancer, or a precancerous stage thereof.

5. *In vitro* use of at least one gene selected from the list consisting of: *AJAP1* and/or *WBSCR17,* or of a kit comprising reagents for the determining the methylation status the genes, for the diagnosis and/or prognosis of pancreatic cancer, or a precancerous stage thereof; and/or monitoring treatment response of patients suffering from pancreatic cancer, or a precancerous stage thereof.

6. *In vitro* use, according to claim 5, which further comprises assessing the methylation status of the gene *PRKCB* in a biological sample obtained from the patient.

7. *In vitro* method, or *in vitro* use, according to any of the claims 2 to 6, which comprises assessing the methylation status of the following gene combinations: *AJAP1 + PRKCB; AJAP1 + WBSCR17; PRKCB + WBSCR17; or AJAP1 + PRKCB + WBSCR17.*

8. *In vitro* method, or *in vitro* use, according to any of the claims 2 to 7, wherein if a deviation of the methylation status is identified, as compared with a pre-established reference value, this is indicative that the biomarker signature may be used for the diagnosis, prognosis or monitoring pancreatic cancer, or a precancerous stage thereof, or is indicative that the subject is suffering from pancreatic cancer, or a precancerous stage thereof, or has a poor prognosis.

9. *In vitro* method, or *in vitro* use, according to any of the claims 2 to 8, wherein the methylation status of the genes is determined in at least a CpG site of the gene.

10. *In vitro* method, or *in vitro* use, according to any of the claims 2 to 9, wherein the methylation status of the genes is determined in at least a CpG site of the promoter region.

11. *In vitro* method, or *in vitro* use, according to any of the claims 2 to 10, wherein the methylation status of the gene *AJAP1* is determined in at least the CpG site cg13495205, wherein the methylation status of the gene *PRKCB* is determined in at least the CpG site cg03306374 and/or wherein the methylation status of the gene *WBSCR17* is determined in at least a CpG site selected from cg03044249 or cg01366419.

12. *In vitro* method, or *in vitro* use, according to any of the claims 2 to 11, wherein the biological sample is selected from: a liquid biopsy selected from plasma, serum, blood, saliva, cerebrospinal fluid or urine, cyst fluid, pancreatic juice or duodenal juice; or a tissue sample

13. Kit, suitable for the diagnosis and/or prognosis of pancreatic cancer, or a precancerous stage thereof; and/or monitoring treatment response of patients suffering from pancreatic cancer, or a precancerous stage thereof, which comprises a pair of primers and a probe for the amplification and detection of at least a fragment of the gene *AJAP1* and/or a pair of primers and a probe for the amplification and detection of at least a fragment of the gene *WBSCR17,* wherein:
a) The forward primer for the amplification of at least a fragment of gene *AJAP1* is selected from SEQ ID NO: 1 or SEQ ID NO: 4;
b) The reverse primer for the amplification of at least a fragment of gene *AJAP1* is selected from SEQ ID NO: 2 or SEQ ID NO: 5;
c) The probe for the detection of the methylation of the gene *AJAP1* is selected from SEQ ID NO: 3 or SEQ ID NO: 6;
d) The forward primer for the amplification of at least a fragment of gene *WBSCR17* is selected from SEQ ID NO: 13 or SEQ ID NO: 16;
e) The reverse primer for the amplification of at least a fragment of gene *WBSCR17* is selected from SEQ ID NO: 14 or SEQ ID NO: 17;
f) The probe for the detection of the methylation of the gene *WBSCR17* is selected from SEQ ID NO: 15 or SEQ ID NO: 18.

14. Kit, according to claim 13, which further comprises a pair of primers and a probe for the amplification and detection of gene *PRKCB,* wherein:
a) The forward primer for the amplification of at least a fragment of gene *PRKCB* is selected from SEQ ID NO: 7 or SEQ ID NO: 10;
b) The reverse primer for the amplification of at least a fragment of gene *PRKCB* is selected from SEQ ID NO: 8 or SEQ ID NO: 11;
c) The probe for the detection of the methylation of the gene *PRKCB* is selected from SEQ ID NO: 9 or SEQ ID NO: 12.
